(19)

Europäisches
Patentamt
European
Patent Office
Office européen
des brevets

(11) **EP 3 135 205 A1**

(12) **EUROPEAN PATENT APPLICATION**

(43) Date of publication:
**01.03.2017  Bulletin 2017/09**

(51) Int Cl.:
*A61B 8/15* (2006.01)　　*A61B 8/08* (2006.01)

(21) Application number: **15182877.9**

(22) Date of filing: **28.08.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(71) Applicant: **ETH Zurich**
**8092 Zurich (CH)**

(72) Inventors:
- **GOKSEL, Orcun**
  **8044 Zürich (CH)**
- **SANABRIA, Sergio**
  **8049 Zürich (CH)**

(54) **HAND-HELD MEDICAL ULTRASOUND APPARATUS**

(57)　A hand-held medical ultrasound apparatus (10), in particular for ultrasound computed tomography of the breast, comprises an ultrasound transducer (1), a reflector (2) and an indicator (311,321, 312) enabling the indication of a relative position and/or orientation between the transducer and the reflector. In a first embodiment, the transducer and the reflector are attached to a mechanical structure comprising a first frame (33) and a second frame (34), which allow to adjust the distance (d) between the transducer and the reflector in order to adapt to the shape of the breast. In a second embodiment, the tranducer and the reflector are not mechanically connected and a position and/or orientation sensor is provided to determine a relative position and/or orientation between the transducer and the reflector. In a third embodiment, thin resonant reflector layers are applied to introduce acoustic signatures in the tracked reflector signals.

FIG. 1

EP 3 135 205 A1

**Description**

Technical Field

The invention is related to a hand-held medical ultrasound apparatus, and to a medical ultrasound system.

Background Art

Tumors and certain other anomalies in breast tissue are not always detectable in conventional B-mode ultrasound systems. However, these pathologies may present high contrast regarding other ultrasound characteristics, such as ultrasound propagation speed and attenuation. Similarly to X-ray Computed Tomography (CT), in order to obtain spatially-resolved images of these parameters, ultrasound waves are transmitted and recorded at multiple angular directions. This presently requires high-end application-specific Ultrasound Computed Tomography (USCT) equipment, based on a large number of stationary ultrasound sensors positioned around the breast, see "Breast density measurements with ultrasound tomography: A comparison with film and digital mammography", Duric et al., Med. Phys. 40(1), January 2013, or by mechanically rotating ultrasound sensors around the breast, see "Imaging of Sound Speed Using Reflection Ultrasound Tomography", Nebeker et al. Ultrasound Med 2012, 31, pages 1389-1404, both of which systems require the immersion of the breast in a water tank and the scanning with a bulky custom-made ultrasound system. Though accurate results, such systems are burdensome in daily clinical use, require additional space in the clinics and specialized personnel to perform, and are typically costly which can only be used for the given specific purpose.

Extensions of current X-ray mammography systems for ultrasound sound-speed and attenuation imaging have been investigated, for example see "Limited-angle ultrasonic transmission tomography of the compressed female breast. Krueger et al. IEEE Ultrasonics Symposium 1998, pages 1345-1348". In this case, the breast is fully compressed between two stationary compression plates, and ultrasound transducers are positioned over and/or below the compression plates. In other embodiments, one of the plates is eliminated and the breast is compressed between a transducer and a stationary plate, see for example "Reconstruction of ultrasonic sound velocity and attenuation coefficient using linear arrays: clinical assessment. Chang et al. 2007 1681-1687". The compression of the breast according to these setups results into a painful diagnosis procedure, similarly to X-ray mammography. It also reduces flexibility, since the ultrasound transducers are either fixed or restricted to move parallel to the compression plates, having only access to coronal planes. Small-sized breast and ultrasound imaging near the chest wall can also prove unfeasible. With respect to USCT equipment, the mammography setup allows only for transmitting and recording through a limited set of angular directions, which leads to strong artifacts in the obtained ultrasound images if the positions and geometry of the anomalies (e.g. tumors) are not known a priori. Moreover, small air gaps are difficult to avoid between the compression plates and the breast, and induce strong artifacts in the ultrasound images. As a consequence, the quality of the diagnoses obtained with these systems is presently poor and none have therefore reached to a commercial implementation level.

Disclosure of the Invention

The problem to be solved by the present invention is therefore to enable a widespread use of ultrasound computed tomography (USCT).

This problem is solved by a hand-held medical ultrasound apparatus, comprising an ultrasound transducer for emitting ultrasound, a reflector for reflecting at least a portion of the emitted ultrasound, and an indicator enabling the indication of a relative position and / or orientation between the transducer and the reflector.

The apparatus not necessarily encompasses a full scale ultrasound computed tomography system, however, the apparatus can be part of and / or connected to such a tomographic unit of such system.

The ultrasound apparatus is a medical apparatus which implies that its use is in a medical context: The apparatus may be used for one or more of medical screening, diagnosis, staging (e.g. of cancer), preoperative planning, intra-operative guidance, and postoperative follow-up.

Hand-held in this context is meant to be portable, or mobile. The apparatus can be held by a sonographer such as a doctor or a nurse during inspecting a patient. Hence, its weight and its extension are dimensioned to apply the apparatus at any place without being bound to a stationary set-up for the transducer.

The ultrasound transducer comprises at least an element for emitting ultrasound, preferably at some frequency in a range between 1 MHz and 40 MHz, and more preferably in a range between 3 MHz and 14 MHz. The ultrasound transducer preferably converts electrical signals into ultrasound waves, e.g. by means of a piezoelectric converter as such element. In a very preferred embodiment, the ultrasound transducer also comprises at least one receiving element, and preferably more, for receiving ultrasound waves, and in particular for receiving reflected ultrasound waves as will be explained below, and for converting the received ultrasound waves into electrical signals.

The apparatus further comprises a reflector for reflecting ultrasound waves emitted by the transducer and travelling through the inspected tissue. Hence, the reflector is of ultrasound reflective property, which may be achieved by choosing the reflector at a different acoustic impedance than the tissue or by applying a material in or on the reflector that is reflective for ultrasound, such as metal (e.g. aluminium, steel), polymers/plastics (e.g. PMMA, Polycarbonate, ABS, rubber, silicone), entrapped air or fluid layers, glass, ceramics, mineral aggregates and other composites or metamaterials.

In operation, it is preferably envisaged that the body tissue to be examined, which preferably is the female breast, is arranged between the transducer and the reflector. It is preferred that the reflector and the transducer are arranged with respect to each other such that the reflector is exposed at least to a part of the ultrasound emitted by the transducer after travelling through the tissue. Preferably, the transducer and the reflector are arranged opposite to each other with the reflector facing directly to or roughly toward the transducer.

Ultrasound waves are sequentially transmitted from the one or more emitting elements, transmitted through the breast target, reflected and / or scattered at the reflector plate and re-acquired by one or more of the receiving elements. This allows for the measurement of ultrasound parameters, in particular ultrasound propagation speed and / or ultrasound attenuation along different angular directions, and allows for the reconstruction of an USCT image in a tomographic unit that the apparatus is connected to. The tomographic unit is understood to convert the signals provided by the apparatus into images to be displayed to the sonographer, for example.

The indicator of the apparatus enables the indication of a relative position and / or orientation between the transducer and the reflector. It is not required to always indicate both the position and the orientation, wherein the position preferably refers to a distance between the transducer and the reflector while the orientation refers to an angle between the transducer and the reflector. One of these two measures may be sufficient, in particular when e.g. the other measure is predefined anyway, e.g. by way of the arrangement of the transducer and the reflector in the apparatus. The indicator not necessarily needs to show the position and / or orientation at the apparatus itself; it may just enable so. In one embodiment, there are provided means at the apparatus itself to derive the positional and / or orientation information in an ad hoc manner by the user. Such means may preferably include a scale or other visual indicators allowing to assess e.g. a distance between the transducer and the reflector. In another embodiment, the apparatus may contain a sensor for one or more of determining the position and / or the orientation. Here, a corresponding sensor signal may be evaluated and the position and / or orientation may be determined in a remote unit such as a tomographic unit to which the sensor signal may be transmitted. In a third variant, the reflector itself may be prepared in a way to allow the identification of the reflector - transducer position / orientation in the image derived from the reflected ultrasound received by the transducer and finally displayed on a display of a tomographic unit.

It is preferred that the apparatus is used in Ultrasound Computed Tomography (USCT) in the medical domain to detect tumorous inclusions in breast tissue, which may not be visible in conventional B-mode images or may be visible but may not be diagnosed or categorized in B-mode images alone. Preferably, the apparatus is prepared to allow a measurement of the speed of ultrasound on its way from the transducer to the reflector and back to the transducer. By transmitting ultrasound waves through tissue between the ultrasound transducer and a reflector of known position and orientation and back through the tissue to the transducer, an USCT image can be obtained. The ultrasound speed can be computed dependent on the length of the path the ultrasound travels, which in the most simple case equals twice the distance between the transducer and the reflector, and dependent on the time taken for travelling this path, which is the time measured between emitting an ultrasound pulse and receiving a reflected portion of the ultrasound pulse. Hence, the present apparatus preferably can be considered as a handheld extension of an USCT tomographic unit.

The present invention provides an apparatus for hand-held and localized breast compression, applicable to USCT, while enabling accurately controlling the positioning and orientation between an ultrasound transducer and a reflector. Most other known breast USCT systems instead require to immerse the breast in a water tank, which adds additional complications in application, whereas the present apparatus system is hand-held, giving it flexibility in use.

Additionally, a standard ultrasound transducer can be employed which is known e.g. from conventional B-mode scanning, in contrast to customized and costly transducer mechanisms of the known systems, which then also allows a clinician to use this transducer for conventional clinical B-mode imaging, by simply decoupling other elements of the apparatus from it.

In an embodiment of the present invention, the transducer and the reflector are attached to or are integral part of a mechanical structure. The transducer and the reflector preferably are arranged opposite to each other. The mechanical structure preferably comprises a distance adjustment for enabling the sonographer to vary the distance between the transducer and the reflector. At least a part of the distance adjustment acts as indicator. Specifically, the mechanical structure comprises a first frame that the transducer is attached to, a second frame that the reflector is attached to or is integrated in or consists of, and at least a first bar both the first and the second frames are mounted to. At least one of the frames is slide-able over the first bar, e.g. by each frame providing a hole into which the bar is inserted. This first bar preferably comprises positioning means for holding the at least one frame at predefined positions such as borings in the first bar. The at least one frame comprises a pin at least partially insertable into the borings one at a time for holding the at least one frame in the predefined position at the first bar. In such embodiment, the pin preferably is mounted in the at least one frame to take a first position reaching into any of the borings, and a second position out of the borings, where the second position is required for sliding the frame between two adjacent borings of the first bar. Preferably, the pin is movable from the first position to the second position against a resilient force. The pin is preferably held into the boring by a spring mechanism adjusted so that the resilient force to achieve a second position can be achieved by hand force. Instead of pin and bores, other releasable adjusting mechanisms such as snap-fits may be used for adjusting the frame to the bar.

However, it may be preferred, for enhancing mechanical stability, that the mechanical structure comprises a second bar with the first frame being mounted to both the first and the second bar and the second frame being mounted to both the first and the second bar. Again, at least one of the frames is slidable mounted, now over both the first and the second bar. Positioning means are now provided at both the first and the second bar for holding the at least one frame at predefined positions. The positioning means preferably include borings at the predefined positions in each of the first and the second bar. The at least one frame comprises a pin at least partially insertable into the borings of the first bar and another pin at least partially insertable into the borings of the second bar for holding the at least one frame in the predefined position.

By attaching or integrating the transducer and the reflector to a hand-held operable mechanical structure, the breast is compressed only locally. The apparatus containing the mechanical structure ensures a fix relative orientation between the transducer and the reflector, provides a direct contact between the transducer and the target, e.g. the breast, preferably reduces the compression area to the active cross-section area of the ultrasound transducer, and allows for hand-held operation, which enables arbitrarily oriented scanning plane and quick adjustment of the reflector distance. Hand-held operation is standard in conventional ultrasound imaging, and is essential for sonographers during examination.

In a second embodiment, a position and / or orientation sensor is provided in the apparatus for allowing to determine a relative position and / orientation between the transducer and the reflector. Preferably, parts of the sensor are attached to both the transducer and the reflector. In one embodiment, a magnetic sensor is used, e.g. including a magnet and a sensing element for sensing a magnetic field. Other technologies, such as optical, electromagnetic, inertial positioning sensing or in general any sensor technology which records relative position and / or orientation while preserving a mostly independent movement between the transducer and the reflector is possible. Once the relative position and / or orientation of the transducer and the reflector fulfill the requirements of USCT imaging, additional misalignments may be compensated for with image processing algorithms based on the sensor information and additional features extracted from the ultrasound measurement. In this second embodiment, it is preferred that the transducer and the reflector are not mechanically connected and can be separately manipulated with respect to the breast target, e.g. with separate hands. However, for example, one or both of the transducer and the reflector may be limited in movement, and e.g. be allowed to move only in a predefined direction and / or orientation.

In a third embodiment, a single or multilayered continuous reflector is used. A single layer may be sufficient since it may allow reflections at both a front and a back side thereof. Thin resonant reflector layers can be applied to introduce acoustic signatures in the tracked reflector signals, which can be separated from reflections observed at undesired structures e.g. within tissue or at air gaps between transducer/target breast/reflector. This allows for cancelling undesired information e.g., from the air interfaces trapped in the ultrasound gel, during an USCT image reconstruction and improves the quality of the reconstructions / imaging. Moreover, thicker reflector layers can be applied to obtain well separated ultrasonic signals from different layers. Under consideration of the layer geometry, the conjoint identification of both separated ultrasound signals provides discrimination of undesired reflective structures. Such layer surface (or thickness) can also be engineered / micro-machined, such as with a frequency ripple pattern, in order to allow for its differentiation in reflection ultrasound images. It should be noted that the reflector geometry is not limited to the presently introduced embodiments, apart from its optionally layered structure. For example, curved reflectors may be envisaged.

Preferably, at least the second frame comprising the reflector, and, if available the first frame comprising the transducer, are of a geometry that does not lead to a full breast compression. Hence, it is preferred that the frame or frames each have a width w and a length 1, wherein the length l may exceed the width w, and wherein the width w of each frame may roughly correspond to the transducer's active cross-section width at least in a region designated for contacting a tissue to investigate, and e.g. be less than 2 cm, and specifically 1 cm or less. Therefore, it is avoided that the full breast is compressed between two plates as may be done in current mammography systems which translates into a painful diagnosis procedure and reduces flexibility. Instead, a relaxed pose of the patient is facilitated during inspection, and a hand-held and localized compression of the breast is achieved, while preserving accurate tracking of the reflector position and / or orientation with respect to the transducer.

Given that in all the embodiments, the relative position and / or orientation is accurately derivable and that the quality of the imaging is highly dependent on an accurate positioning and orientation between the transducer and the reflector, images of excellent quality can be achieved. Furthermore, compared to a mammography setup, the transducer is not restricted to move along a compression plate, having only access to coronal planes. Instead, the transducer is hand-operated and in direct contact with the breast, which enables flexible access to arbitrary breast positions and orientations. In the same context, small air gaps between the prior art compression plate and the breast can be avoided. These air gaps introduce strong artifacts in the USCT images. And, small-sized breasts and ultrasound imaging near the chest wall are now facilitated for accommodation compared to previous compression plate systems.

In summary, breast compression now is limited to a cross-section of the ultrasound transducer, which significantly reduces the subject pain related to the diagnosis. Arbitrary orientation and positioning of the transducer with respect to the breast is enabled, which provides similar flexibility to the sonographer for USCT compared with a conventional hand-operated B-mode transducer. Small air gaps between compression plate and breast are minimized by reducing the compression area. Moreover, remaining air inclusions can be identified and removed from the images by profiting from the layered structure of the reflector if available.

The presented invention provides a low-cost hand-held alternative to state-of-the-art high-end ultrasound tomography systems. Conventional B-mode systems can be used for USCT with a minor addition of passive mechanical components plus dedicated software. The present apparatus can be used as an add-on to conventional B-mode ultrasound equipment, particularly for breast cancer detection. However, the invention also allows for the detection and differentiation of other anomalies of the subject tissue such as lesion / fibradenoma / cysts, also giving information about size and / or depth and / or location.

Apart from breast scanning, other applications and targets may be envisaged, in which the described test geometry is applicable, e.g. in medical imaging for finger / leg / arm scanning, or in general for non-destructive testing of materials, biological or non-biological. Furthermore, other applications of a reproducible positioning of a reflector with respect to an ultrasound transducer for tomographic imaging may be found in medical imaging or even for non-destructive testing of material properties, e.g. soft or deformable solid materials, such as foams.

Other advantageous embodiments are listed in the dependent claims as well as in the description below.

Brief Description of the Drawings

The embodiments defined above and further embodiments, features and advantages of the present invention can also be derived from the examples to be described hereinafter and are explained with reference to the annexed drawings, wherein:

FIG. 1 illustrates a diagram of an apparatus according to an embodiment of the present invention;
FIG. 2 illustrates a diagram of an apparatus according to another embodiment of the present invention;
FIG. 3 illustrates a diagram of an apparatus according to a third embodiment of the present invention;
FIG. 4 illustrates a block diagram of a system according to an embodiment of the present invention;
FIG. 5 shows details of the embodiment of Figure 1;
FIG. 6 shows an apparatus in a perspective view in diagram 6a), and in application to a mimic breast in diagram 6b), according to an embodiment of the invention, the apparatus of FIG. 6 preferably coinciding with the apparatus schematically shown in FIG. 1;
FIG. 7 shows an apparatus in a perspective view in an application to a mimic breast, according to an embodiment of the invention, the apparatus of FIG. 7 preferably coinciding with the apparatus schematically shown in FIG. 2;
FIG. 8 shows details of the embodiment of Figure 3;
FIG. 9 shows a reflector arrangement of an apparatus, in an exploded view in diagram 9a), and in an assembled view in diagram 9b), according to an embodiment of the present invention, which reflector arrangement may specifically be used in the apparatus shown in FIG. 3;
FIG. 10 shows sample tomographic images reconstructed according to a data evaluation proposed according to an embodiment of the present invention; and
FIG. 11 shows sample tomographic images reconstructed according to a data evaluation proposed according to an embodiment of the present invention.

Detailed Description of the Drawings

Same elements are referred to by the same reference signs across all Figures.

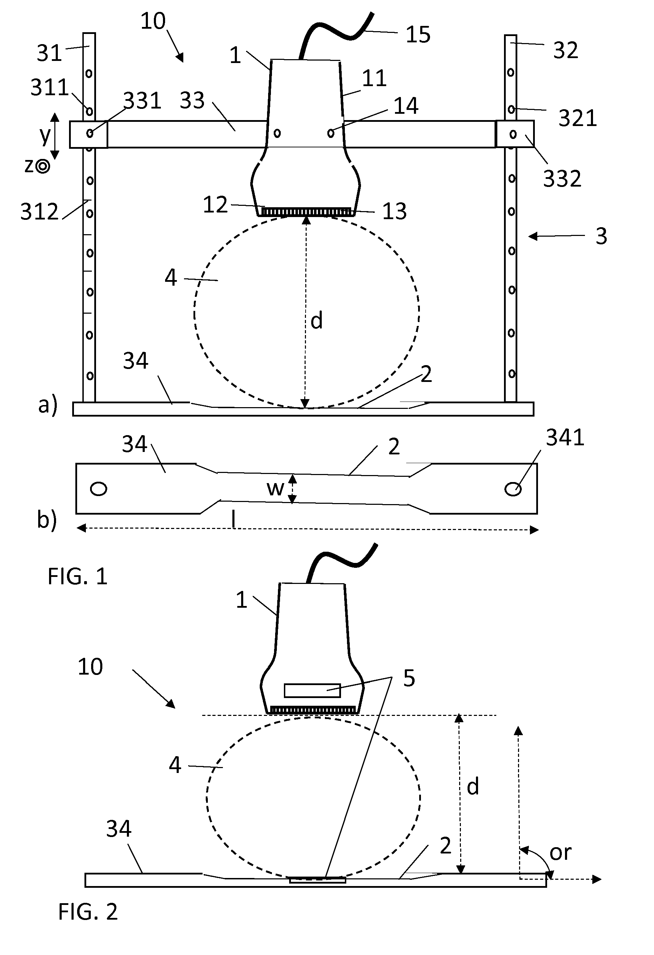

Figure 1a) illustrates a side view of a hand-held medical ultrasound apparatus 10 according to a first embodiment of the present invention. The apparatus 10 comprises an ultrasound transducer 1 and a reflector 2. The transducer 1 and the reflector 2 are arranged opposite to each other. In between, a target to be investigated is indicated by reference numeral 4, i.e. a female breast in the present example. Ultrasound waves emitted by an array of ultrasound emitters 12 travel through the tissue of the breast 4 and at least a portion thereof is reflected by the reflector 2. The transducer 12 further comprises an array of ultrasound receivers 13 for receiving reflected ultrasound waves and converting these into electrical signals. Ultrasound emitters 12 and receivers 13 can be formed by a common array as is indicated in Figure 1.

The transducer 1 comprises a housing 11, which is fixed to a first frame 33 by means of fixing means 14 such as screws. If screw holes are not available in the transducer, the fixing means 14 can be a plastic mold that accurately reproduces the transducer geometry. The mold can be manufactured e.g. with a 3D printing device for an arbitrary commercial transducer geometry. The transducer is then inserted and fixed into the plastic mold. The transducer 1 preferably is connected via a cable 15 to a tomographic unit, preferably a conventional medical ultrasound system (not shown) and is configured to send electrical signals representing the received ultrasound waves thereto, or signals derived therefrom.

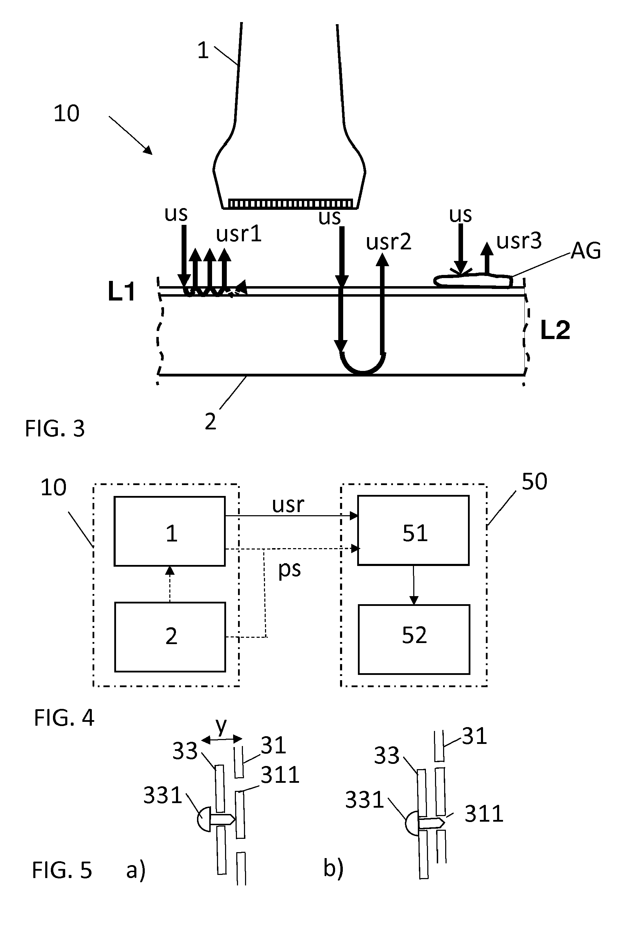

The first frame 33 is made from rigid material such as metal or plastics. The first frame 33 is slidable mounted along the y-axis over a first bar 31 and a second bar 32. The first and the second bar 31, 32 are each made from rigid material such as metal or plastics, and preferably take a cylindrical hollow shape. Each of the first and the second bar 31, 32 comprises bores 311, 321 preferably arranged equidistant as positioning means for the first frame 33. The first frame 33 comprises at each of its ends a pin 331, 332 that is capable of being at least partially inserted into one of the bores 331, 332. Each pin 331, 332 may e.g. be a bolt, a screw, or other element as long as it is insertable into a bore of the bars 31, 32. Hence, bores 311 and pin 331 together provide a means for holding a left end of the first frame 33 in a defined position, while bores 321 and pin 322 together provide a means for holding a right end of the first frame 33 in a defined position. In case the pins 331 and 332 are not inserted in any of the bores 311, 321 the first frame 33 is movable along the y-axis between two adjacent borings of each bar 31, 32. This scenario is shown in Figure 5a) in a cut-out and with respect to pin 331. Instead, Figure 5b) illustrates a scenario in a cut-out in which the pin 331 is inserted in one of the bores 311. The pin 331 is movable in z-direction. Preferably, the pins 331 and 332 are mounted in the first frame 33 against a resilient force, for instance, a spring mechanism, which makes the respective pin enter a bore once crossing it. For releasing the first frame 33 from a bore, the two pins 331 and 332 are lifted and slid in z-direction against the respective resilient force, e.g. manually, for allowing the first frame 33 to become movable again along the bars 31 and 32. Hence, the first frame 33 including the transducer 1 can be hand-operated vertically slid towards a second frame 34 including the reflector 2. The resilient force is sufficient high to keep the two frames 33 and 34 stable with respect to the target 4 once the position has been adjusted, but small enough to be released by hand when the frame must become movable again. Additional elements, such as a clamping ring, maybe used to stabilize the frame 33 in a defined pin position.

At their bottom end, the two bars 31 and 32 are attached to the second frame 34, preferably welded, screwed or otherwise mounted, either releasable or non-releasable. In the present example, a distance d between the transducer 1 and the reflector 2 can be adjusted by moving the first frame 33 relative to the second frame 34. In another embodiment, the second frame 34 may be additionally slidable over the two bars 31 and 32 in the same manner as is the first frame 33, e.g. by providing corresponding pins at the end of the second frame 34. In a different embodiment, the first frame 33 is fixed in its position with the bars 31 and 32, and only the second frame 34 comprising the reflector 2 is slidable over the bars 31 and 32.

Hence, frames 33 and 34 as well as bars 31 and 32 contribute to a mechanical structure 3 for holding the transducer 1 and the reflector 2, and for both allowing the distance d between the transducer 1 and the reflector 2 be varied / adjusted, and for determining a distance adjusted between the transducer 1 and the reflector 2. For supporting this purpose, one or both of the bars 31, 32 may be provided with a scale 312 allowing the sonographer to read, estimate or deduct the distance d or this distance may be read by a sensor automatically. Hence, the pin/bore-mechanism acts as a distance adjuster which on the one hand allows the fixing of a defined compression thickness by manually sliding the first frame 33 towards the second frame 34 until a release point defined by the pins entering one of the borings. The compression preferably is released by simply sliding the first frame 33 upwards. No screw loosening or tightening is necessary during this process. In one embodiment, there is not even a scale required but the sonographer can determine the distance solely by e.g. the number of free bores between the two frames 33, 34 together with the knowledge of a distance between adjacent bores.

Diagram 1b) illustrates a top view on the second frame 34 of the apparatus shown in Figure 1a). At its ends, mounting holes 341 are provided for mounting the second frame 34 to the bars 31 and 32, e.g. for welding at these very locations. The second frame 34 has a length l and a width w, which width w is defined at a location of the second frame 34 that is expected to touch the target, i.e. the breast 4. The second frame 34 may be of uniform width, or may be of varying width along its length l as is shown in Figure 1b). The width w preferably roughly corresponds to the transducer's active cross-section width, which is typically less than 2 cm, preferably equal to or less than 1 cm. Hence, the second frame 34 is not a compression plate but serves for only locally compressing the breast. Preferably, the first frame 33 is of a similar width at the location of compression such that the localized compression concept is not impeded.

For taking ultrasound readings of the breast 4, the sonographer preferably moves the first frame 33 in a direction in and out of the plane of projection, thereby possibly adjusting the distance d between the transducer 1 and the reflector 2 for adapting to the shape of the breast. The sonographer may at each position record an ultrasound image which may be assembled and visualized by the tomographic unit connected to the cable 15.

The reflector 2 may be one of attached to the second frame 34, be integrated therein, or be represented by the second frame 34. E.g. in the latter case, the second frame 34 may be entirely of metal and act as a reflector 2. In a different embodiment, reflector material may be attached, e.g. be adhered to the second frame 34 which in this case may not be manufactured from an ultrasonic reflecting material but may be made e.g. from plastics.

Figure 2 illustrates a side view of a hand-held medical ultrasound apparatus 10 according to a second embodiment of the present invention. The apparatus 10 comprises an ultrasound transducer 1 which may be identical to the transducer 1 of Figure 1, and a reflector 2 which may be identical to the reflector of Figure 1. The transducer 1 and the reflector 2 are arranged opposite to each other and the target to be investigated is arranged, and preferably slightly compressed in between. However, no bars are provided for providing mechanical stability and a defined distance d and / or a defined orientation between the transducer 1 and the reflector 2. However, in different embodiments, one or both or the transducer and the reflector may be mounted to allow a movement in only a defined direction or orientation. For example, the reflector may be pivot mounted at one of its end and therefore only change its position by way of rotating. In the present embodiment, a position and / or orientation sensor 5 is provided. Such sensor 5 may determine either the distance d between the transducer 1 and the reflector 2, or the orientation or there between, or preferably both. The sensor 5 may comprise elements arranged at both, the transducer 1 and the reflector 2. The sensor 5 is built based on medically approved technologies. For example, for magnetic position tracking, the sensor 5 includes a base for inducing a strong magnetic field and small receiver coils for reading the induced field. The base and the receiver coils are all connected (cabled) to the same unit to deduce position. In this case, receiver coils may be arranged at both the reflector 2 and the transducer 1. Another possibility is to use optical tracking, e.g. with infrared or visible lights, by arranging passive or active markers at both the reflector 2 and the transducer 1. Or, subject to the sensing principle, the sensor 5 may be arranged only at one of the transducer 1 and the reflector 2.

Figure 3 illustrates a side view of a hand-held medical ultrasound apparatus 10 according to a third embodiment of the present invention. The apparatus 10 comprises an ultrasound transducer 1 which may be identical to the transducer 1 of Figure 1, and a reflector 2. The transducer 1 and the reflector 2 are arranged opposite to each other and the target to be investigated is arranged in between (not explicitly shown in Figure 3). Again, no bars are provided for providing mechanical stability between the transducer 1 and the reflector 2. Instead, the reflector 2 comprises a two-layered set-up including a second layer L2 with second reflection properties, and a first layer L1 on top of the second layer L2 with first reflection properties with respect to ultrasound, which second reflection properties are different to the first reflection properties. Hence, a first portion of the ultrasound us emitted is reflected by the layer L1 and is received as reflected ultrasound signal usr1 by the receiver in the transducer 1. Another portion of the ultrasound us emitted is reflected by the second layer L2 and is received as reflected ultrasound signal usr2 by the receiver in the transducer 1. The thickness of layer L1 is thin enough to induce acoustic signatures in the tracked reflector signals, for example the cancellation or enhancement of determined ultrasound frequencies. The thickness of layer L2 is large enough to obtain well separated ultrasonic signals usr2 and usr1. Both layers L1 and L2 provide complementary discrimination means to cancel reflections usr3 at undesired structures, for instance an air gap AG between tissue and reflector 2. These discrimination means can therefore be used individually, for instance, the reflector can consist only of layer L1 or L2, or combined for better discrimination. Additional layers may be added if necessary.

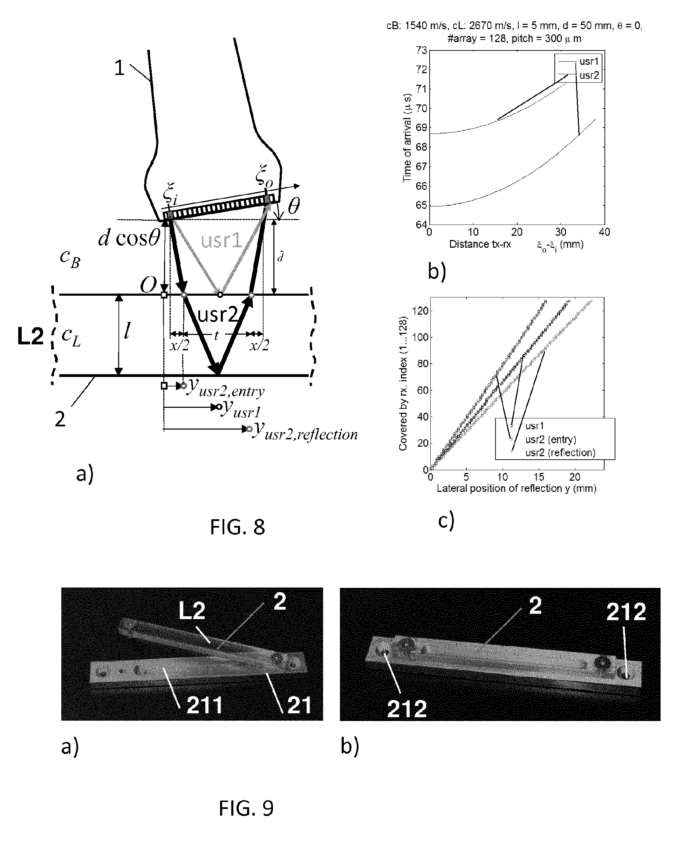

Figure 8 illustrates a particular embodiment, in which only a single reflector layer L2 is used. An arbitrary wave propagation path between an emitter element $\xi_i$, also referred to as transmitter element, of the transducer 1 and a receiver element $\xi_o$ of the transducer 1 is considered, which elements $\xi_i$, $\xi_o$ may be arbitrary transducer element pairs. The transducer 1 is separated by an unknown distance d from the reflector 2, which is inclined by an unknown angle $\theta$ with respect to the transducer 1. $cB$ is the unknown average ultrasound propagation speed in the breast tissue medium between the transducer 1 and the reflector 2 (not shown). The thickness $l$ and the average ultrasound propagation speed $cL$ in the layer L2 are known. The measured time of arrival t1, t2 of the ultrasound reflection signals at the top usr1 and bottom usr2 interfaces of the reflector 2 are functions of the unknown parameters. For reflection at the top usr1 surface, the time of arrival t1 is calculated as follows:

$$t_1 = c_B^{-1} \sqrt{4d^2 + \left(\xi_o - \xi_i\right)^2 + 4\sin^2\theta\left(\xi_i\xi_o - d^2\right) - 2\sin(2\theta)d\left(\xi_o + \xi_i\right)}$$

Note that if $\theta = 0°$ the equation reduces to:

$$t_1^2 = \left(c_B^{-2}\right)\left(4d^2 + \left(\xi_o - \xi_i\right)^2\right)$$

which can be optimized for both d and $c_B$ with linear least squares optimization. The former equation is however not linear and must be solved with a non-linear optimization approach, preferably Nelder-Mead simplex optimization or any other appropriate method.

For reflection at the bottom surface usr2,

$$t_2 = c_B^{-1}\sqrt{4\hat{d}^2 + x^2} + c_L^{-1}\sqrt{4l^2 + (t-x)^2} + c_B^{-1}\sqrt{[t\cos\theta - (\xi_o - \xi_i)]^2 + [t\sin\theta]^2}$$

$$t = (\xi_o - \xi_i)(\cos\theta - \sin\theta)x/(2\hat{d})$$

$$p_4 x^4 + p_3 x^3 + p_2 x^2 + p_1 x + p_0 = 0$$

$$p_4 = (c_B^{-2} - c_L^{-2})[1 + (\xi_o - \xi_i)\sin\theta/(2\hat{d})]^2$$

$$p_3 = -2(\xi_o - \xi_i)(c_B^{-2} - c_L^{-2})[1 + (\xi_o - \xi_i)\sin\theta/(2\hat{d})]\cos\theta;$$

$$p_2 = c_B^{-2}[4l^2 + (\xi_o - \xi_i)^2\cos^2\theta] - c_L^{-2}[4\hat{d}^2 + 4\hat{d}\sin\theta(\xi_o - \xi_i) + (\xi_o - \xi_i)^2]$$

$$p_1 = 8(\xi_o - \xi_i)c_L^{-2}\hat{d}^2[1 + (\xi_o - \xi_i)\sin\theta/(2\hat{d})]\cos\theta$$

$$p_0 = -4\hat{d}^2 c_L^{-2}(\xi_o - \xi_i)^2\cos\theta$$

$$\hat{d} = (d + \tan\phi\,\xi_i)\cos\theta$$

the time of arrival t2 is calculated by solving a 4th degree polynomial:

where $\xi_i$ is the known transmitter lateral position, $\xi_i$ is the known receiver lateral position, d is the unknown distance between transducer and plate with respect to the first transducer element, $\theta$ is the unknown inclination between transducer and plate, $cB$ is the unknown average ultrasound propagation speed in the inspected medium 4, $l$ is the known thickness of the plate L2 and cL is the known average ultrasound propagation speed in the plate.

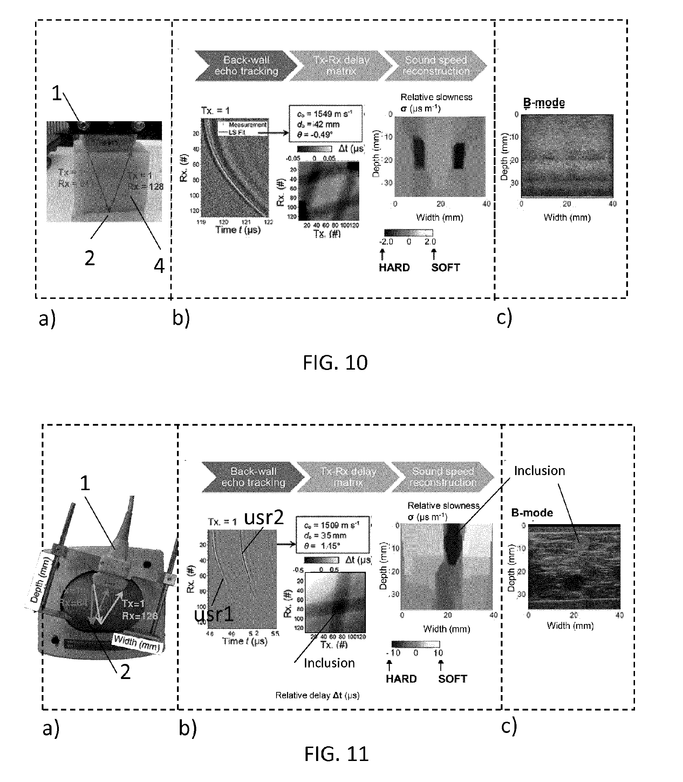

Figure 8b) shows the reflection delays calculated for a preferred configuration, for the transmitter element $\xi_i$, and in which layer L2 is a 5 mm thick Plexiglas plate (cL = 2670 m/s). With the chosen material parameters, a controlled and approximately constant delay between the two reflections usr1 and usr2 is achieved (~3 μs), which is a compromise between the time discrimination of successive ultrasound reflection signals, and the signal-to-noise ratio of the second echo usr2 which is the lower the thicker the reflector L2 is. Experimental results are shown in Figure 11b).

Figure 8c) illustrates the applicability of simultaneous detection of the two echoes usr1, usr2 to improve the robustness of the reflector tracker, again for the first transducer element $\xi_i$. For each lateral position $y$ of the reflector 2 there is at least a receiver position corresponding to an ultrasound signal usr1 reflected at such position, as well as two receiver positions for ultrasound signals usr2 respectively incident and reflected at the position $y$. The three signals should be simultaneously detectable for the same position $y$ and moreover provide consistent time estimates t1 and t2 according to the equations shown above. Therefore, the simultaneous consideration of multiple reflection signals in a non-linear optimization algorithm can be used to improve the accuracy of the reflection timing and to filter out reflections usr3 at undesired structures, as shown in Figure 3. Outlier detection algorithms, for instance Random sample consensus (RANSAC) can be used to filter out such undesired structures from the measured time matrices. The equations provided above are simplified equations, which assume straight ray trajectories between transducer elements $\xi$ and reflector 2. In a more general implementation, these equations are refined iteratively until convergence with a full-wave solution that accounts for refraction, diffraction and scattering phenomena within the inhomogeneous tissue medium. Similarly, other wave signatures apart from the time delays (ultrasound attenuation, linear frequency response, non-linear effects) can be used for reflector tracking and tomography reconstruction with the presented embodiments.

Figure 4 illustrates a block diagram of a system according to an embodiment of the present invention. The system comprises a portable apparatus 10 according to any one of the preceding embodiments, and a stationary tomographic unit 50 remote from the apparatus 10. A transducer 1 of the apparatus transmits electrical signals representing the reflected ultrasound waves usr to a processing unit 51 of the tomographic unit 50, where the signals usr are evaluated and preferably converted into cut view images of the target. The resulting images preferably are displayed on a display 52 of the tomographic unit 50. In case the distance and / or orientation between the transducer 1 and the reflector 2 is sensed by a corresponding sensor in the apparatus 10, it is preferred that this position information ps is transmitted to the processor unit 51, too, either by the transducer 1 or by the reflector 2, subject to the set-up of the sensor.

The tomographic unit 50 may in one embodiment be based on a commercial FDA-approved research ultrasound machine, e.g. a SonixTablet/SonixTouch, Ultrasonix Medical Corporation, Richmond, BC, Canada. Such machine provides a programming interface by means of which user-defined ultrasound acquisition sequences can be defined. Similar machines are available in the market from other manufacturers, e.g. Verasonics Inc., Kirkland, WA, USA; SuperSonic, Aix-en-Provence, France. According to a preferred embodiment, for the present ultrasound tomography, the emitter and receiver array of the transducer 1 is typically operated in multi-static mode, with each element individually firing and the rest receiving. This concept is illustrated in Figure 10a), and is equivalent to ultrasound imaging with transmitter and receiver aperture of one element. In order to improve the signal-to-noise ratio when transmitting through thick breast tissue, a larger transmitted aperture, typically 2 or 4 elements, may be used. In general, any clinically approved ultrasound machine, which allow for definition of both the transmitter and receiver aperture, may also be used for ultrasound tomography according the presented invention, as long as the reflected echoes usr1, usr2 are sampled with sufficient temporal resolution. Radiofrequency data RF is therefore in general preferred, even if B-mode images may also be used for reflector detection.

Generally, and irrespective of any of the above embodiments, in particular the distance as determined between the transducer 1 and the reflector 2 may be used in determining a speed the ultrasound travels through the target which speed may indicate tissue irregularities. And / or, the position and / or orientation between the transducer 1 and the reflector 2 may be used for identifying the relevant areas in the cut image taken by the target.

Hence, and in general, a reflector based total-variation sound-speed imaging and delineation of piecewise homogeneous inclusions in breast tissue is proposed in one embodiment, without the requirement of knowing a position of the inclusion in advance. For example, a 128-emitting and receiving element array in the transducer is operated in a multistatic mode, each element individually firing and the rest receiving. Preferably, adaptive amplitude-tracking measures the delays of echoes reflected from the reflector behind the sample. Dynamic programming algorithms or other algorithms based on graph theory or random Markov fields, among others, may also be used to track the delays of echoes in a continuous fashion. Non-linear optimization of the $128 \times 128$ delay matrix, for instance Nelder-Mead simplex optimization and/or RANSAC outlier filtering, provides average sound speed, plate distance and inclination, together with relative delays $\Delta t$ induced by sound speed inhomogeneities. With a known geometric path-length L (i.e. the distance between the transducer and the reflector), relative slowness increments $\sigma$ (low: high sound speed/hard inclusion; high: low sound speed/soft inclusion) are preferably solved from an ill-conditioned linear system $\Delta t = L\sigma$. The total-variation regularization $\arg\min_\sigma \{\|\Delta t - L\sigma\|\_2 + \lambda\|D\ \sigma\|\_1\}$, with D a gradient matrix, is preferably solved with convex optimization. The same equation structure or an iteratively adjusted version of it can be used for solving for other wave signatures, such as ultrasound attenuation or other linear or non-linear features.

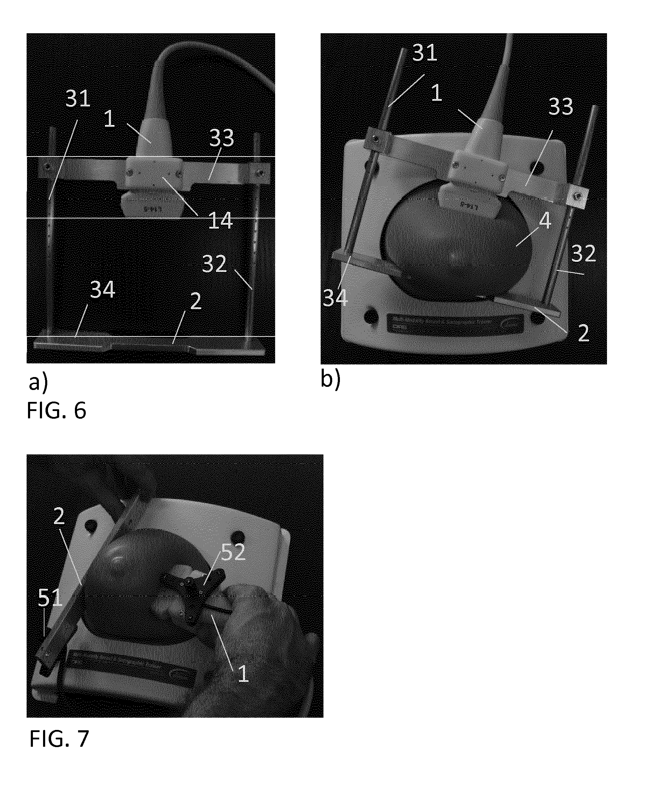

Figure 6 shows an apparatus in a perspective view in diagram 6a), and in application to a mimic breast in diagram 6b), according to an embodiment of the invention, the apparatus of Figure 6 preferably coinciding with the apparatus schematically shown in Figure 1, such that the following disclosure shall in particular also be applicable to the embodiment of Figure 1. The ultrasound transducer 1 preferably is a commercial linear array (L14-5, Ultrasonix Medical Corporation, Richmond, BC, Canada). It may comprise a total of 128 transducer elements, which can alternatively act as transmitters or receivers, with a pitch between elements of 300 $\mu$m, an element elevation of 7 mm, and a total aperture of 38 mm. The transducer 1 provides two-dimensional ultrasound imaging in a plane perpendicular to the transducer elements, with the width of the image corresponding to the linear array direction, and the depth corresponding to the perpendicular direction to the transducer elements, along which ultrasound echoes are recorded in function of time, see Figure 1 and Figure 11. Other transducers types, for example convex array ultrasound probes or two-dimensional ultrasound arrays can be similarly used.

The reflector 2 preferably is an aluminum plate with a width w of 10 mm at the target contact region, which is arranged opposite to the transducer 1. Figure 6b) shows the same implementation during the inspection of an ultrasound phantom (Model 059, Computerized Imaging Reference Systems, Inc (CIRS), Norfolk, VA, USA), which mimics a female breast 4. The fixing means 14 is here a plastic mold of the transducer geometry (polycarbonate) manufactured with 3D printing technology. Both first 33 and second frame 34 are made from aluminum, with second frame 34 acting simultaneously as reflector 2. The bars 31 and 32 are cylindrical and massive and are fabricated with stainless steel. The bores are 90° countersink borings machined into the bars 31,32. They are arranged equidistant as positioning reference with steps of 5 or 10 mm. The pin is a screw with a ball bearing tip, which is attached to the second frame 34 with a nut. The ball bearing is attached with a string to the screw (pressure screw) and provides an adequate resilient force for hand-held fixing and releasing the first frame 33.

Figure 7 shows an apparatus in a perspective view in application to a mimic breast, according to an embodiment of the invention, the apparatus of FIG. 6 preferably coinciding with the apparatus schematically shown in Figure 2, such that the following disclosure shall in particular also be applicable to the embodiment of Figure 2. The transducer 1, reflector 2 and breast phantom 4 are the same as in Figure 6. The transducer 1 and the reflector 2 can be moved freely and independently with separate hands. An optic sensor includes passive and active markers 51 and 52 which are attached to both transducer 1 and reflector 2, and allows real time tracking of the relative displacement and orientation between each other (Easytrack 500, Atracsys, Puidoux, Switzerland). The sonographer first searches for a region of interest by moving the transducer 1 along the breast 4, preferably receiving real time feedback in terms of B-mode ultrasound images on a display of an ultrasound tomography unit. Once a desired position has been identified, the reflector 2 is moved by hand until it is roughly aligned opposite to the transducer 1. Both elements are pressed slightly onto the breast, preferably with a coupling agent (e.g. water, ultrasound gel, honey, oil) in between, in order to achieve a good acoustic coupling. The optic sensor 5 provides a real time feedback on the display 52 and informs when the alignment is good enough to perform tomographic imaging. Moreover it provides geometrical referencing of the transducer 1 and reflector 2 with respect to the breast phantom, allowing the construction of volumetric breast scans by successive acquisition of ultrasound image planes at known positions and orientations.

Figure 9 shows a reflector arrangement used in an apparatus according to an embodiment of the present invention, which reflector arrangement may specifically be used in an apparatus as shown in Figure 3, such that the following disclosure shall in particular also be applicable to the embodiment of Figure 3. The reflector 2 may include the specific material geometry as is calculated in connection with Figure 8b). In contrast to Figure 3, a single 5 mm thick, w = 100 mm wide reflector layer L2 is used as reflector 2. The reflector 2 is made from Plexiglas™ (cL = 2670 m/s, rhoL = 1200 kg/m$^3$), which shows a good acoustic contrast with tissue (cB = 1540 m/s, rhoB = 1000 kg/m$^3$). A top surface of the layer L2 is in contact with the inspected breast tissue, providing a reflection coefficient (with normal incidence) for the reflected ultrasound signal usr1 of R = (cL*rhoL - cB*rhoB)/(cL*rhoL + cB*rhoB) = 0.35 for usr1. A bottom surface of L2 is in contact with air (cAir = 346 m/s, rhoAir = 1.2 kg/m$^3$), providing a strong reflection coefficient of opposite sign for the reflected ultrasound signal usr2, R = (cAir*rhoAir - cL*rhoL)/(cAir*rhoAir + cL*rhoL) = -0.9997. For a reproducible reflection behavior of usr2, the bottom surface of layer L2 preferably is kept clear. With this purpose, a plate 21 is attached below the reflector layer L2, with an engraving 211 that ensures an interface Plexiglas-air in the region of interest. The mounted reflector arrangement is shown in Figure 9b), and may additionally be attached through screw holes 212 to e.g. the second frame 34.

Figure 10 shows an ultrasound tomography example according to a data evaluation proposed according to an embodiment of the presented invention. A gelatin phantom 4 containing two 5 mm cylindrical inclusions is inspected with a transducer 1, with <1% ultrasound propagation speed contrast, see Figure 11a). The inclusions do not show echogenicity contrast with respect to the background and are therefore invisible in B-mode images, which corresponding image is shown in Figure 11c). A large plate of a reflector 2 with a single reflecting interface is tracked. An adaptive amplitude-tracking described above successfully measures the delays of echoes reflected from the reflector 2 behind the sample, see Figure 11b). Nelder-Mead simplex optimization of the $128 \times 128$ delay matrix achieves a least-square (LS) fit of the time profiles according to the multi-static wave trajectories, and provides average sound speed $c_o$, plate distance $d_o$ and inclination $\theta$ of the reflector 2. The average sound speed $c_o$ has already diagnostic value, since a dense breast, which is more prone to certain pathologies, shows higher sound speed than the average breast. The proposed limited-angle reconstruction with total-variation regularization does not suffer from strong streaking artifacts as observed in previous art. On the contrary, the two inclusions are sharply and piecewise smoothly delineated in the reconstructed sound speed image, see Figure 11b). No prior knowledge about the position or geometry of the inclusions is necessary. A loss of resolution along the depth may be suffered, which is inherent to all limited-angle approaches due to missing projection information in quasi-parallel directions to the reflector plate. As a consequence, an approximately 150% elongation of circular inclusions is observed inside square phantoms, which still provides an excellent inclusion discrimination.

Figure 11 demonstrates the application of the presented hand-held apparatus according to an embodiment of the present invention to cancerous mass detection. A breast phantom is investigated by an apparatus according to Figures 6 or 1, see Figure 12a). The reflector system illustrated in Figure 9 is used. Two well-separated ultrasound echoes, usr1 and usr2, are obtained at the reflector 2, which allows for a robust reflection tracking as described in Figure 8, see Figure 12b). The second reflection signal usr2 shows opposite sign with respect to the first reflection signal usr1, due to the negative reflection coefficient in the interface Plexiglas-air. This reflection configuration allows a very robust tracking of the ultrasound signals, and achieves a high-quality time delay matrix $\Delta t$, see Figure 12b). In comparison, it is clearly observed that measurements with wide reflector plates and a single reflector layer, as performed in the previous art mammography setups (Krueger et al. 1998, Chang et al 2007), result in significantly noisier time delay matrices $\Delta t$, due to the presence of uncontrolled air gaps and due to the presence of spurious out-of-plane propagation paths. Hence, the apparatus described in the presented invention, apart from achieving higher diagnosis flexibility and reducing diagnosis pain, provides a clear cut improvement of the quality of the recorded ultrasound signals.

The reconstructed sound speed image shows a strong and localized increase of speed of sound by about 5% (decrease of slowness), revealing a stiff inclusion at width = 25 mm, depth = 12 mm, see Figure 12b). A stiff inclusion is representative of a cancerous tumor, and is therefore of highest interest in ultrasound breast diagnosis. A B-mode image of the corresponding area, see Figure 12c) provides an indication of heterogeneity at this position, but does not provide conclusive diagnostic feedback about its nature. For example, other suspected masses between depths 20 and 30 mm in the B-mode image show much lower stiffness contrast, indicating their cystic nature, and are not revealed in the B-mode image. The total-variation regularization approach provides a piecewise smooth ultrasound image, with an excellent contrast between stiff inclusion and background. Therefore, the apparatus is capable of providing high-quality tomographic images in which tumorous inclusions can be delineated, allowing the differentiation of such inclusions from benign cystic masses.

## Claims

1. Hand-held medical ultrasound apparatus (10), comprising
   an ultrasound transducer (1) for emitting ultrasound,
   a reflector (2) for reflecting at least a portion of the emitted ultrasound, and
   an indicator (21,22,311,312) enabling the indication of a relative position and / or orientation between the transducer (1) and the reflector (1).

2. Apparatus (10) according to claim 1,
   wherein the transducer (1) and the reflector (2) are attached to a mechanical structure (3) opposite to each other.

3. Apparatus (10) according to claim 2,
   wherein the mechanical structure (3) comprises a distance adjustment for varying a distance (d) between the transducer (1) and the reflector (2), at least a part of the distance adjustment acting as indicator (311,312).

4. Apparatus (10) according to claim 2 or claim 3,
   wherein the mechanical structure (3) comprises a first frame (33) the transducer (1) is attached to, a second frame (34) the reflector (2) is attached to or is integrated in or consists of, and at least a first bar (31) both the first and the second frame (33,34) are mounted to, and
   wherein at least one of the frames (33) is slidable mounted over the first bar (31).

5. Apparatus (10) according to claim 3 or claim 4,
   wherein the first bar (31) comprises positioning means for holding the at least one frame (33) at predefined positions.

6. Apparatus (10) according to claim 5,
   wherein the positioning means includes borings (311) at the predefined positions in the first bar (31),
   wherein the at least one frame (33,34) comprises a pin (331) at least partially insertable into the borings (311) for holding the at least one frame (31) in the predefined position at the first bar (31).

7. Apparatus (10) according to claim 6,
   wherein the pin (331) is mounted in the at least one frame (33) to take a first position reaching at least partially into any of the borings (311), and a second position out of the borings (311) which second position is required for sliding the frame (33) between two adjacent borings (311) of the first bar (31), and
   in particular wherein the pin (331) is movable from the first position to the second position against a resilient force.

8. Apparatus (10) according to claim 4,
   wherein the mechanical structure (3) comprises a second bar (32),
   wherein the first frame (33) is mounted to both the first and the second bar (31,32),
   wherein the second frame (34) is mounted to both the first and the second bar (31,32),
   wherein at least one of the frames (33) is slidable mounted over both the first and the second bar (33, 34),
   wherein each of the first and the second bar (31,32) comprises positioning means for holding the at least one frame (33) at predefined positions.

9. Apparatus (10) according to claim 8,
wherein the positioning means includes borings (311,321) at the predefined positions in each of the first and the second bar (31,32), and
wherein the at least one frame (33) comprises a pin (311) at least partially insertable into the borings (311) of the first bar (31) and another pin (321) at least partially insertable into the borings (321) of the second bar (32) for holding the at least one frame (33) in the predefined position.

10. Apparatus (10) according to any one of the preceding claims,
wherein the indicator includes a position and / or an orientation sensor (5) for determining a position and / or an orientation respectively between the transducer (1) and the reflector (2), and
in particular wherein the position and / or the orientation sensor (51) is a magnetic or optic sensor.

11. Apparatus according to claim 10,
wherein the transducer (1) and the reflector (2) are mechanically disconnected, and
in particular wherein the reflector (1) is attached to or is integrated in or consist of a planar frame (34).

12. Apparatus according to any one the preceding claims,
wherein the indicator includes the reflector (2), which reflector (2) comprises layers (21,22) of different ultrasound reflecting properties.

13. Apparatus according to any one the preceding claims,
wherein the first frame (33) and the second frame (34) each have a width (w) and a length (1), the length (1) exceeding the width (w),
wherein the width (w) of each frame (33, 34) is less than 2 cm at least in a region designated for contacting a tissue to investigate.

14. Medical ultrasound system, comprising

   - an apparatus according to any of the preceding claims,
   - a processor (51),

wherein the transducer (1) is electrically connected to the processor (51), and
wherein the processor (51) is configured to determine an ultrasound based tomographic image subject to reflected ultrasound waves (usr) received by the ultrasound transducer (1).

15. Medical ultrasound system according to claim 14,
wherein the processing unit is configured to apply total-variation regularization in the calculation of tomographic ultrasound images, and
in particular wherein in the total variation regularization the equation to be solved follows the form $\mathrm{argmin}_\sigma \{\|\Delta t - L\sigma\|_2 + \lambda\|D \sigma\|_1\}$ or any combination of such forms, where $\Delta t$ is a vector of measured quantities, $\sigma$ the unknown vector to be reconstructed, L a matrix geometrically calculated under consideration of the setup geometry, D a gradient matrix and $\lambda$ a constant, and
in particular wherein the equation is solved with convex optimization.

FIG. 1

FIG. 2

FIG. 3

FIG. 4

FIG. 5    a)                    b)

a)

FIG. 6

FIG. 7

FIG. 8

FIG. 9

FIG. 10

FIG. 11

Europäisches
Patentamt

European
Patent Office

Office européen
des brevets

# EUROPEAN SEARCH REPORT

Application Number

EP 15 18 2877

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHANG ET AL: "Reconstruction of Ultrasonic Sound Velocity and Attenuation Coefficient Using Linear Arrays: Clinical Assessment", ULTRASOUND IN MEDICINE AND BIOLOGY, vol. 33, no. 11, 26 October 2007 (2007-10-26), pages 1681-1687, XP022317511, ISSN: 0301-5629, DOI: 10.1016/J.ULTRASMEDBIO.2006.10.013 | 1-9,13, 14 | INV. A61B8/15 A61B8/08 |
| Y | * the whole document * | 15 | |
| X | WO 2010/029556 A1 (SLENDER MEDICAL LTD [IL]; AZHARI HAIM [IL]; TSOREF LIAT [IL]; GROSS YO) 18 March 2010 (2010-03-18) | 1-11,13, 14 | |
| Y | * the whole document * | 15 | |
| Y | CUIPING LI ET AL: "Breast ultrasound tomography with total-variation regularization", PROCEEDINGS OF SPIE, vol. 7265, 1 January 2009 (2009-01-01), pages 726506-1-726506-8, XP055031262, DOI: 10.1117/12.811044 * sections 2.1 and 2.2 * | 15 | |
| A | EP 1 281 354 A2 (ERMERT HELMUT PROF DR-ING [DE]; ASHFAQ MOHAMMAD DIPL-ING [DE]; HILTAWS) 5 February 2003 (2003-02-05) * the whole document * | 1,14 | TECHNICAL FIELDS SEARCHED (IPC) A61B |
| A | US 2006/116579 A1 (LI PAI-CHI [TW] ET AL) 1 June 2006 (2006-06-01) * the whole document * | 1,14 | |

-/--

The present search report has been drawn up for all claims

| Place of search | Date of completion of the search | Examiner |
|---|---|---|
| Munich | 18 February 2016 | Küster, Gunilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 18 2877

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HANSEN C ET AL: "Reconstruction of Speed of Sound for a Correction of Transit Time in Full Angle Spatial Compounding", 2007 IEEE ULTRASONICS SYMPOSIUM, 1 October 2007 (2007-10-01), pages 785-788, XP031195092, DOI: 10.1109/ULTSYM.2007.201 ISBN: 978-1-4244-1383-6 * sections I and II.A., figs. 1-3 * | 1,14 | |

TECHNICAL FIELDS
SEARCHED      (IPC)

The present search report has been drawn up for all claims

| Place of search | Date of completion of the search | Examiner |
|---|---|---|
| Munich | 18 February 2016 | Küster, Gunilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 18 2877

This annex lists the patent family members relating to the patent documents cited in the above-mentioned European search report.
The members are as contained in the European Patent Office EDP file on
The European Patent Office is in no way liable for these particulars which are merely given for the purpose of information.

18-02-2016

| Patent document cited in search report | | Publication date | Patent family member(s) | | Publication date |
|---|---|---|---|---|---|
| WO 2010029556 | A1 | 18-03-2010 | NONE | | |
| EP 1281354 | A2 | 05-02-2003 | DE | 10137186 A1 | 20-02-2003 |
| | | | EP | 1281354 A2 | 05-02-2003 |
| US 2006116579 | A1 | 01-06-2006 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DURIC et al.** Breast density measurements with ultrasound tomography: A comparison with film and digital mammography. *Med. Phys.,* January 2013, vol. 40 (1
- **NEBEKER et al.** Imaging of Sound Speed Using Reflection Ultrasound Tomography. *Ultrasound Med,* 2012, vol. 31, 1389-1404
- **KRUEGER et al.** Limited-angle ultrasonic transmission tomography of the compressed female breast. *IEEE Ultrasonics Symposium,* 1998, 1345-1348
- **CHANG et al.** *Reconstruction of ultrasonic sound velocity and attenuation coefficient using linear arrays: clinical assessment,* 2007, 1681-1687